# EUROPEAN PATENT APPLICATION

(11) **EP 4 840 388 A1**
(43) Date of publication of application: **18.11.2026**
(21) Application number: 25176168.0
(22) Date of filing: 13.05.2025
(51) Int. Cl.: A61B 5/00, A61B 5/11, A61B 5/16, G06F 3/01, G06V 10/70, A61B 5/055

(54) **TRIGGERING AN ALERT USING A SUBJECT SPECIFIC MOTION PATTERN**

(71) Applicant: Koninklijke Philips N.V., 5656 AE Eindhoven (NL)
(72) Inventor: HELLE, Michael Günter, Eindhoven (NL); WUELBERN, Jan Hendrik, Eindhoven (NL); NAUTS, Sanne, 5656AG Eindhoven (NL)
(74) Representative: Philips Intellectual Property & Standards

(57) **Abstract**

Disclosed herein is a method of operating a medical imaging system (100, 300, 500) configured for acquiring medical imaging data (134, 134') descriptive of a subject (108) on a subject support (106). The medical imaging system comprises a remote motion sensing system (112, 112') configured for acoustically, visually, or electromagnetically acquiring subject motion data (138) descriptive of motion of the subject on the subject support. The method comprises: receiving (200) a selection of a subject specific motion pattern (132); acquiring (202) the medical imaging data using the medical imaging system; continually (204) receiving the subject motion data during the acquisition of the medical imaging data; continually (206) searching the motion data for the subject specific motion pattern using a pattern recognition algorithm; and triggering (208) an alert (142) if the subject specific motion pattern is detected.

## Description

### FIELD OF THE INVENTION

The invention relates to medical imaging, in particular to receiving a signal from a subject during a medical imaging procedure.

### BACKGROUND OF THE INVENTION

In medical imaging systems such as Magnetic Resonance Imaging (MRI) systems, a nurse call mechanism can serve as a component for generating a signal or alert in response to specific system conditions, such as patient discomfort, hardware anomalies, or safety-related events. In MRI systems specifically, a commonly used mechanism is a squeeze ball, which allows the patient to manually initiate an alert by applying pressure. This input is translated into a signal or alert that is transmitted to the operator of the medical imaging system so that the operator can intervene on behalf of the subject being imaged.

United States patent application US2020008703A1 discloses a method for monitoring a patient during a medical imaging examination, at least one state signal of the patient is detected using at least one state detector, and the at least one detected state signal of the patient is evaluated using an evaluation algorithm. An emotional state of the patient can be established based on the detected state signal. The method can further include generating output information based on the established emotional state of the patient and outputting the output information via an output interface.

### SUMMARY OF THE INVENTION

The invention provides for a method of operating a medical imaging system, a computer program product, and a medical imaging system in the independent claims. Embodiments are given in the dependent claims.

In one aspect a method of operating the medical imaging system is disclosed. The medical imaging system is configured for acquiring medical imaging data descriptive of a subject on a subject support. The medical imaging system comprises a remote motion sensing system configured for acoustically, visually, and/or electromagnetically acquiring motion data descriptive of motion of a subject on the subject support. The method comprises receiving a selection of a subject specific motion pattern. The method further comprises acquiring the medical imaging data using the medical imaging system. The method further comprises continually receiving the subject motion data during the acquisition of the medical imaging data. The method further comprises continually searching the motion data for the subject specific motion pattern using a pattern recognition algorithm. The method further comprises triggering an alert if the subject specific motion pattern is detected. The alert preferably activates a subject communication system configured for opening or operating a communication channel between the subject and an operator of the medical imaging system.

In another aspect a computer program product is disclosed. The computer program product may in particular be a computer-readable medium. The computer program product carries machine-executable instructions for execution by a computational system controlling a medical imaging system. Execution of the machine-executable instructions causes the computational system to perform an example of the method of operating the medical imaging system.

In another aspect a medical imaging system is disclosed. The medical imaging system is configured for acquiring medical imaging data descriptive of a subject on a subject support. The medical imaging system comprises a remote sensing system configured for acoustically, visually, or electromagnetically acquiring motion data descriptive of motion of a subject on the subject support. The medical imaging system further comprises a memory storing machine-executable instructions. The medical imaging system further comprises a computational system. Execution of the machine-executable instructions causes the computational system to receive a selection of a subject specific motion pattern. Execution of the machine-executable instructions further causes the computational system to acquire the medical imaging data using the medical imaging system. Execution of the machine-executable instructions further causes the computational system to continually receive the subject motion data during the acquisition of the medical imaging data. Execution of the machine-executable instructions further causes the computational system to continually search the motion data for the subject specific motion pattern using a pattern recognition algorithm. Execution of the machine-executable instructions further causes the computational system to trigger an alert if the subject specific motion pattern is detected. The alert preferably activates a subject communication system configured for opening a communication channel between the subject and operator of the medical imaging system.

### BRIEF DESCRIPTION OF THE DRAWINGS

In the following preferred embodiments of the invention will be described, by way of example only, and with reference to the drawings in which:
Fig. 1 illustrates an example of a medical imaging system.
Fig. 2 shows a flow chart which illustrates a method of using the medical imaging system of Fig. 1.
Fig. 3 illustrates a further example of a medical imaging system which incorporates a magnetic resonance imaging system.
Fig. 4 illustrates the use of visual markers within the medical imaging system of Fig. 3.
Fig. 5 illustrates a further example of a medical imaging system which incorporates a cloud based computing system.

### DETAILED DESCRIPTION OF EMBODIMENTS

Like numbered elements in these figures are either equivalent elements or perform the same function. Elements which have been discussed previously will not necessarily be discussed in later figures if the function is equivalent.

In one aspect a method of operating a medical imaging system is disclosed. The medical imaging system is configured for acquiring medical imaging data descriptive of a subject on a subject support. In various examples the medical imaging data may be data which may be used to reconstruct an anatomical or internal image or map of the subject. For example, the medical imaging data may be k-space data and may be used for reconstructing a magnetic resonance image. In other examples, the medical imaging data could be a so-called CT profile and used to reconstruct a computed tomography image. The medical imaging system comprises a remote motion sensing system.

The remote motion sensing system may for example be used to acoustically, visually, or electromagnetically acquire motion data descriptive of motion of a subject on the subject support. The remote motion sensing system may be configured to detect or measure the motion of the subject on the subject support from a distance. For example, acoustically may encompass the use of sound or sonar data or ultrasound to detect motion of the subject. Visually could encompass the use of optical instruments also in the ultraviolet or infra-red for measuring direct motion of the subject. Electromagnetically may encompass such remote sensing techniques as radar or Doppler radar. Regardless of the mechanism the medical imaging system uses a remote sensing system to acquire motion data descriptive of motion of the subject on the subject support. This may for example indicate the position of the limbs or other body parts of the subject such as the positioning or gaze of the eyes or expression or position of a subject's face.

The method comprises receiving a selection of a subject specific motion pattern. The subject specific motion pattern may be a pattern of motions which the subject consciously decides to perform. The subject specific motion pattern may for example then be used as a signal to the medical imaging system. The method further comprises acquiring the medical imaging data using the medical imaging system. This may encompass acquiring the medical imaging data to use to later reconstruct into a medical image. The method further comprises continually receiving the subject motion data during the acquisition of the medical imaging data. So as the medical imaging data is being acquired the subject motion data is also acquired or received. The method further comprises continually searching the motion data for the subject specific motion pattern using a pattern recognition algorithm.

The pattern recognition algorithm may use algorithmic techniques or may also include such things as using a neural network to recognize certain movements or motion patterns. The method further comprises triggering an alert if a subject specific motion pattern is detected. This example may be beneficial because the specific sequence of subject motion patterns were used to trigger the alert. This means that the subject may use this as a signal in cases where the subject may not be able to communicate or trigger the alert otherwise. For example, if a subject has a disability or other medical condition and is unable to move their hands or squeeze a ball, which is typically used in magnetic resonance imaging to trigger an alert, then the performing of the subject specific motion patterns may be used to trigger the alert. This for example may provide for greater safety when using the medical imaging system.

In medical imaging procedures such as MRI and CT exams, patients may face challenges in communicating distress or discomfort during the procedure due to the limitations of traditional nurse call buttons. Conventional call systems, such as pressing a ball filled with air in the case of MRI examinations, may not be suitable to activate for patients with limited mobility or with communication impairments (e.g. due to rheumatism, paralysis etc.) and may struggle to alert operators or staff about their needs.

The conventional nurse call buttons used in medical imaging devices may be unsuitable for subject undergoing, for example, MRI or CT scans. Subjects are often unable to utilize traditional call systems in these confined spaces, particularly if their mobility or communication abilities are limited. The lack of an effective communication method can lead to delays in addressing patient distress or discomfort, potentially compromising the quality of the imaging procedure and the patient's overall experience. It is important to introduce communication methods that present high reliability and can be easily activated by patients while at the same time result in minimized false-positives, i.e. that patients unintentionally call the operators as this would distract the workflow and prolong imaging procedure times. The use of the subject specific motion pattern may ensure that the triggering of the alert by the subject is an intentional act.

In some examples there may be optical or Camera-Based Monitoring to provide the subject motion data. These examples may include the usage of cameras to observe and interpret patient actions and facial expressions. This includes lip reading, facial recognition for expressions and eye blinking patterns, as well as tracking predefined motion patterns of body parts (e.g. extremities) to identify distress or communication attempts.

In further examples the remote motion sensing system may be configured to measure eye tracking and body gestures. Examples may facilitate communication through eye tracking capabilities, allowing patients to indicate a desire to communicate with staff by focusing on a specific visual marker within the imaging device.

In addition to the remote sensing, the subject motion data may be augmented in some examples by measuring physiological parameters. Additionally, the subject's heart rate and breathing data can be monitored to enhance the accuracy of distress detection.

In some further examples, subject motion data from different modalities is combined. Since this approach may be suitable for patients with mobility or communication impairments ideally these methods may be used in mutual combination to be applicable to a maximum variety of patients.

In various examples, there may be modifications that avoid or reduce unintentional triggering of the alert. These include
a. Pre-calibration/training performed individually per patient or patient group to distinguish certain motion patterns (e.g. tremor, movements triggered by motor tics) from goal-directed motion aiming to activate the nurse call.
b. Adapting the trigger to a specific gesture or movement pattern an individual patient is able to perform taking into account their movement capabilities, which may be limited by illness or other conditions. For example, paralyzed patients may only be able to communicate by blinking, hemiplegia stroke patients may only be able to move one arm, patients with an injured arm that is less functional should focus on the arm that is functional; patients with Tourette's should use their non-dominant hand/arm to trigger the nurse call, etc.
c. Instead of training a model, one can select from a limited set of pre-defined options. Choose the best option manually or by using information from the exam card, electronic patient record, or camera image analysis.
d. In an advanced approach, a self-learning system can be used for continuous improvement of the alarm triggering, e.g. by implementing a feedback loop that analyses if a certain movement leads to an increased number of false-positive alarms (or false-negatives) and require replacement by a better approach. One method to provide feedback to the system is by manual input of the operator, e.g. after an alarm was triggered.

A further example could include an adjustable discrimination threshold such that the operator or other staff can adjust the specificity to sensitivity trade-off individually. This could include determining how closely the subject motion data matches the subject specific motion pattern.

Examples may provide an alternative to the traditional nurse call button used during medical imaging examinations. This may be important for patients that are not able to use the nurse call button to initiate communication or alert the radiology operator. Various examples may utilize camera-based patient observation, physiology monitoring, or any combination of these to initiate the communication between patient and operator.

In another example the alert preferably activates a subject communication system configured for opening a communication channel between the subject and operator of the medical imaging system. This example may be particularly beneficial because often times it may be difficult to determine when to open the communication channel. For example, if one were to simply look at the expression or mood of a subject by looking at the face of the subject there may be false positives when using the pattern recognition algorithm to determine if the communication channel should be opened. If the subject performs the subject specific motion pattern then it can be determined that the alert was triggered intentionally by the subject. This may enable a greater control of the medical imaging system as well as providing for better safety.

In another example the pattern recognition algorithm comprises a trained neural network. As was mentioned above, subject motion data may be acquired through one or more sensing modalities, including a camera, an ultrasound system, or a radar system. The collected data is processed by a neural network that has been trained to recognize motions which a subject may consciously perform. This may enable the identification of intentional motion signatures, which may be used for monitoring. Trained neural networks may be used for detecting subject specific motion patterns. Subject motion data is acquired through one or more sensing modalities, including a camera, an ultrasound system, or a radar system. The collected data is processed by a neural network that has been trained to recognize motion characteristics unique to individual subjects. This may enable the identification of personalized motion signatures, which may be used for monitoring, alerting, or adapting system behavior based on the subject's activity or state.

The sensing modalities operate independently or in combination to capture various types of motion data. When using a camera, the system can interpret full-body gestures, eye movement, and facial expressions. The ultrasound system provides motion tracking through acoustic signal reflection, while radar enables motion detection through electromagnetic wave analysis, including micromovements. These sensing systems provide input to the neural network in real-time or from recorded sessions, allowing for continuous monitoring.

The neural network model may be trained using labeled data corresponding to known motion patterns for one or more individuals. Through supervised or unsupervised learning techniques, the system develops the capability to distinguish motion patterns that are characteristic of a specific subject. The output of the neural network may be used to trigger alerts, personalize system responses, or integrate with other components in a larger monitoring or diagnostic framework.

Various types of neural network architectures may be employed to implement the motion pattern detection system, depending on the nature and dimensionality of the input data. Convolutional Neural Networks (CNNs) can be used for analyzing spatial data from video frames, particularly for detecting facial expressions, eye motion, or body gestures captured by a camera. Recurrent Neural Networks (RNNs), including Long Short-Term Memory (LSTM) networks, are well-suited for modeling temporal sequences and can be used to learn motion patterns over time from radar or ultrasound data. Transformer-based architectures may also be applied for modeling complex temporal dependencies, especially in multimodal input scenarios. These networks can be trained individually or as part of an ensemble to enhance robustness and accuracy in detecting subject specific motion signatures, alerting, or adapting system behavior based on the subject's activity or state.

The sensing modalities operate independently or in combination to capture various types of motion data. When using a camera, the system can interpret full-body gestures, eye movement, and facial expressions. The ultrasound system provides motion tracking through acoustic signal reflection, while radar enables motion detection through electromagnetic wave analysis. These sensing modalities may provide input to the neural network in real-time or from recorded sessions, allowing for continuous or event-driven analysis.

The neural network model may be trained using labeled data corresponding to known motion patterns for one or more individuals. Through supervised or unsupervised learning techniques, the system develops the capability to distinguish motion patterns that are characteristic of a specific subject. The output of the neural network may be used to trigger alerts, personalize system responses, or integrate with other components in a larger monitoring or diagnostic framework.

Various types of neural network architectures may be employed to implement the motion pattern detection system, depending on the nature and dimensionality of the input data. Convolutional Neural Networks (CNNs) can be used for analyzing spatial data from video frames, particularly for detecting facial expressions, eye motion, or body gestures captured by a camera. Recurrent Neural Networks (RNNs), including Long Short-Term Memory (LSTM) networks, are well-suited for modeling temporal sequences and can be used to learn motion patterns over time from radar or ultrasound data. Transformer-based architectures may also be applied for modeling complex temporal dependencies, especially in multimodal input scenarios. These networks can be trained individually or as part of an ensemble to enhance robustness and accuracy in detecting the subject specific motion pattern.

In another example the pattern recognition algorithm comprises an algorithmic pattern recognition algorithm. This example may be beneficial because the pattern recognition algorithm may be fine-tuned to the particular subject specific motion pattern.

In another example the algorithmic pattern recognition module is any one of the following: a Gaussian mixture model, an optical flow algorithm, a feature tracking algorithm, a clustering algorithm operating on an outputted optical flow algorithm, and combinations thereof. As an alternative to neural networks, the above-mentioned classical algorithms and statistical models can be used to detect subject specific motion patterns from sensor data. A Gaussian Mixture Model (GMM) can model the distribution of motion features by representing complex data as a combination of multiple Gaussian distributions. GMMs are particularly useful for identifying clusters or patterns within motion data, such as typical movement regions or frequently occurring postures, which can be associated with specific subjects. Similarly, clustering algorithms-such as k-means or DBSCAN-can group similar motion trajectories or features, potentially identifying unique patterns across multiple observations without requiring labeled data.

Optical flow algorithms estimate motion between video frames by computing the apparent movement of brightness patterns. These methods are effective in camera-based systems, where they can track fine-grained body movements, facial expressions, or eye motion. The output of an optical flow algorithm can serve as input to higher-level models or be analyzed directly using feature tracking algorithms, which follow distinct visual points across frames to construct motion trajectories. These trajectories may then be processed by a clustering algorithm to identify and group common motion patterns. Combinations of these approaches can be implemented to leverage their complementary strengths-for example, using optical flow to generate motion vectors, clustering to extract recurring behaviors, and GMMs to model the statistical characteristics of those behaviors-thereby enabling robust subject specific motion analysis without deep learning.

In another example the subject specific motion pattern comprises a sequence of discreet subject motions. This may be beneficial because the subject may be told to perform the discreet subject motions in order to trigger the alert. This, for example, may be communicated in a way in which the subject does not need to be trained to perform the discreet subject motions.

In another example the method further comprises acquiring pre-acquisition subject motion data descriptive of the subject using the remote motion sensing system and then pre-calibrating or training the pattern recognition algorithm using the pre-acquisition subject motion data. The pre-calibrating or training being adapted to minimize false positive identification of the subject specific motion pattern by the pattern recognition algorithm. In this example the pattern recognition algorithm is fine-tuned or trained before use. For example, the subject can be placed onto the subject support and told the subject specific motion pattern and then told to repeat it before the beginning of the acquisition of the medical imaging data. In this way the algorithm can be fine-tuned to the subject's exact motions or performance of the subject specific motion pattern. For example, if the system were using eye tracking and the subject were told to look at a certain sequence of images or locations then the system could have its eye tracking of the subject calibrated well.

In another example the method further comprises selecting the subject specific motion pattern from a predetermined list of subject motions. Instead of having the possible subject motions openended there may be a predetermined list and then various elements of this list are selected one or more times to provide the subject specific motion pattern. This may have the benefit that the pattern recognition algorithm only needs to recognize the specific motions or actions which are on the predetermined list.

In another example the method further comprises labeling the subject motion data if the alert was correct or a false positive. The method further comprises training or adjusting the pattern recognition algorithm using the labeled subject motion data. This may be particularly useful if the pattern recognition algorithm is a trained neural network. This for example may be used to generate and produce training data which is then used to improve the quality of the pattern recognition algorithm, or in particular, a neural network.

In another example the remote motion sensing system comprises a camera system. The camera system may, for example, comprise one or more imaging devices. The subject motion data comprises optical images. The use of a camera system may provide a very flexible and effective means of acquiring the subject motion data. Using a camera or optical system, the optical images may be able to record and interpret complex or compound motions of the subject.

In another example the subject specific motion pattern comprises any one of the following: lip reading data, predetermined facial expressions, eye expressions, blinking patterns, and combinations thereof. These for example may include motions with which a person who is handicapped or paralyzed in some capacity may perform. This may for example enable the triggering of an alert when it was not possible before. The lip reading data could for example be used to detect a certain phrase being vocalized by the subject without the use of acoustic data. This may for example be particularly useful in a magnetic resonance imaging system where the environment is noisy. The subject could also be coached to perform the subject specific motion pattern as a sequence of predetermined facial expressions, eye expressions, blinking patterns, and combinations thereof. This may provide for a very effective means of triggering the alert intentionally.

In another example the camera system is configured for eye tracking. The subject specific motion data comprises eye motion data. The subject specific motion pattern comprises eye motion data descriptive of looking at a predetermined location. The predetermined location is preferably a visual marker or predetermined location on a video screen. This embodiment may provide for extremely complex communication without the use of motion of the subject's body or speech. By looking at a predetermined location or a sequence of predetermined locations the subject can trigger the alert. This may for example be by a visual marker or location which is mounted on the inside of a magnetic resonance imaging tube. Alternatively, one could have a video screen and by selecting different icons or locations on the video screen the subject could piece together fairly complex subject specific motion patterns by only moving their eyes. This may also provide for a very secure method of triggering the alert without false positives.

In another example the remote motion sensing system comprises any one of the following: a radar, an ultrasound imaging system, a heart rate monitor, a breathing rate monitor, and combinations thereof. The radar and ultrasound image may for example be useful in tracking the gross motion of the subject. The heart rate monitor and breathing rate monitor may be used in combination to monitor the physiological state of the subject in addition to just monitoring the subject specific motion pattern. If the subject has an abnormal heart rate or breathing rate then this may be used to enhance the correct triggering of the alert by detecting when the subject is in a distressed physiological state.

In another example the medical imaging system comprises a magnetic resonance imaging system configured for acquiring at least a portion of the medical imaging data. This example may be particularly beneficial because there is some risk during magnetic resonance imaging that the subject could have RF heating due to radio-frequency transmissions made. Typically, a squeeze ball is used by the subject to trigger an alert. However, in some instances, the subject may lack the physical coordination or may be incapable of using a hand ball to trigger the alert. Examples may provide an enhanced means of detecting when the subject is in distress or needs to communicate with the operator even when acoustic communication is impaired or is not possible due to the loud noise of the gradient coils in the magnetic resonance imaging system.

In another example, the medical imaging system comprises any one of the following for acquiring at least a portion or a further portion of the medical imaging data: a positron emission tomography system, a single-photon emission tomography system, a computed tomography system, a digital fluoroscope, a digital X-ray system, and combinations thereof. In some instances, one or more of these imaging modalities may also be combined with a magnetic resonance imaging system.

In another example there is a computer program product which may comprise a computer readable storage medium. The computer program product carries or embodies machine-executable instructions for execution by a computational system controlling an example of the medical imaging system. Execution of the machine-executable instructions causes the computational system to perform the method according to an example. The advantages of this have been previously discussed.

In another aspect a medical imaging system is disclosed. The medical imaging system is configured for acquiring medical imaging data descriptive of a subject on a subject support. The medical imaging system comprises a remote motion sensing system configured for acoustically, visually, or electromagnetically acquiring motion data descriptive of a motion of a subject on the subject support. The medical imaging system further comprises a memory storing machine-executable instructions. The medical imaging system comprises a computational system. The execution of the machine-executable instructions causes the computational system to receive a selection of a subject specific motion pattern. Execution of the machine-executable instructions further causes the computational system to acquire the medical imaging data using the medical imaging system. Execution of the machine-executable instructions further causes the computational system to continually receive the subject motion data during the acquisition of the medical imaging data. Execution of the machine-executable instructions further causes the computational system to continually search the motion data for the subject specific motion pattern using the pattern recognition algorithm. Execution of the machine-executable instructions further causes the computational system to trigger an alert if the subject specific motion pattern is detected. The alert preferably activates a subject communication system configured for opening a communication channel between the subject and operator of the medical imaging system. The benefits of this medical imaging system have been previously discussed.

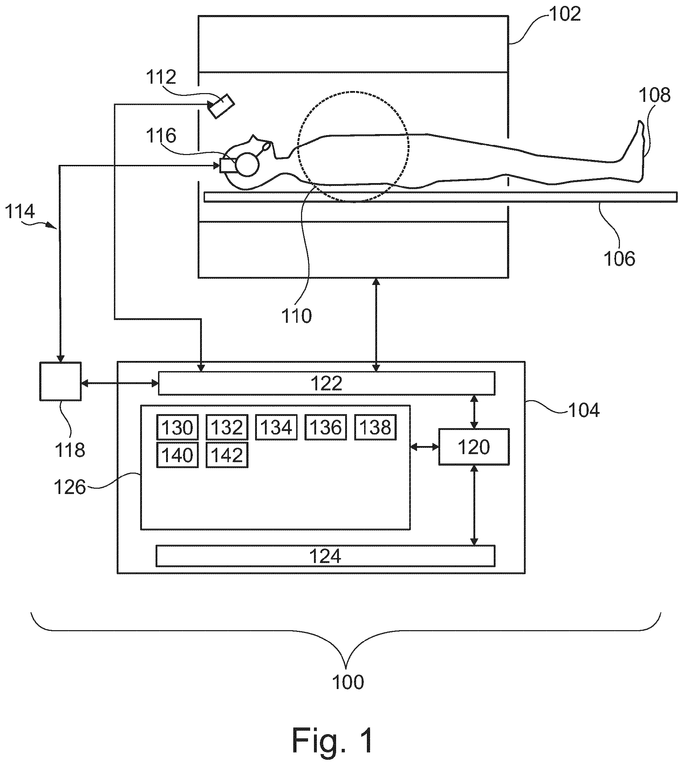

Fig. 1 illustrates an example of a medical imaging system 100 that comprises a medical scanner 102 and a computer 104. The computer 104 may function as a local controller for the medical scanner 102. The medical scanner 102 is intended to be representative and may for example be a magnetic resonance imaging system, a computed tomography system, a positron emission tomography system, a single-photon emission tomography system, a digital X-ray system, a digital fluoroscope or other medical imaging modality. There is a subject support 106 that supports a subject 108 at least partially within a medical imaging zone 110. The medical imaging zone 110 represents the volume from which medical imaging data can be acquired. The medical imaging data is used to reconstruct medical images. There is a remote motion sensing system 112 that may for example use acoustic, visual, or electromagnetic energy and sensing to acquire motion data descriptive of motion of the subject 108. In many embodiments the remote motion sensing system 112 may be a camera and used to observe motions made by the subject 108. There is an optional subject communication system 114 that comprises a subject speaker and microphone 116, and an operator speaker and microphone 118. The subject communication system 114 provides a communication channel. It may for example not always be active and may be triggered or engaged by the computer 104.

The computer 104 comprises a computational system 120. The computational system 120 and the computer 104 are intended to be representational. The computer 104 may represent one or more computing systems at one or more locations and the computational system 120 may represent one or more computing cores or computational systems at one or more locations. The computational system 120 is shown as being in communication with a hardware interface 122 that may be used to control the medical scanner 102 as well as control the remote motion sensing system 112 and the optional subject communication system 114.

The computational system 120 is further in communication with an optional user interface 124 that may enable an operator to use and control the medical imaging system 100. The computational system 120 is further shown as in communication with a memory 126. The memory 126 represents various types of memory which the computational system 120 may have access to. The memory 126 is shown as storing machine-executable instructions 130. The machine-executable instructions 130 enable the computational system 120 to perform such tasks as controlling the medical scanner 102 as well as performing numerical and image processing tasks. The memory 126 is shown as having a subject specific motion pattern 132 that has been stored or selected. These are a sequence of motions which the subject 108 may consciously make as a signal. The memory 126 is further shown as containing medical imaging data 134. The medical imaging data 134 is acquired from the medical imaging zone 110. The memory 126 is further shown as containing a pattern recognition algorithm 136. The memory 126 is further shown as containing subject motion data 138 that was acquired using the remote motion sensing system 112.

The memory 126 is further shown as containing a current motion pattern 140 that was obtained by inputting the subject motion data 138 into the pattern recognition algorithm 136. The current motion pattern 140 can be compared to the subject specific motion pattern 132. If this is the case then an alert 142 may be issued. The alert may control various actions such as activating the subject communication system 114. In other instances an audio or visual alarm may also be provided. In some instances it may also cause the medical scanner 102 to stop.

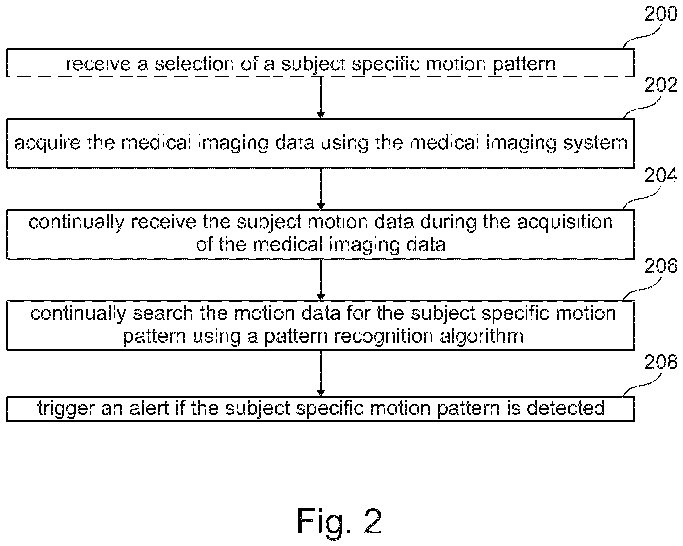

Fig. 2 shows a flowchart which illustrates a method of operating the medical imaging system 100 of Fig. 1. In 200, the selection of the subject specific motion pattern 132 is received. In 202, the medical imaging data 134 is received using or controlling the medical imaging system 100. Specifically, this may be the control of the medical scanner 102 using control commands. For example if the medical scanner 102 were a magnetic resonance imaging system, the medical imaging data would be k-space data and the control commands would be a pulse sequence. In 204, the subject motion data 138 is continually received during the acquisition of the medical imaging data 134. In 206, the subject motion data 138 is continually searched for the subject specific motion pattern using the pattern recognition algorithm 136. In some instances this could be performed by generating the current motion pattern 140 from the subject motion data 138 or in other instances it may be performed directly by analyzing the subject motion data 138. In 208, the alert 142 is triggered if the subject specific motion pattern 132 is detected.

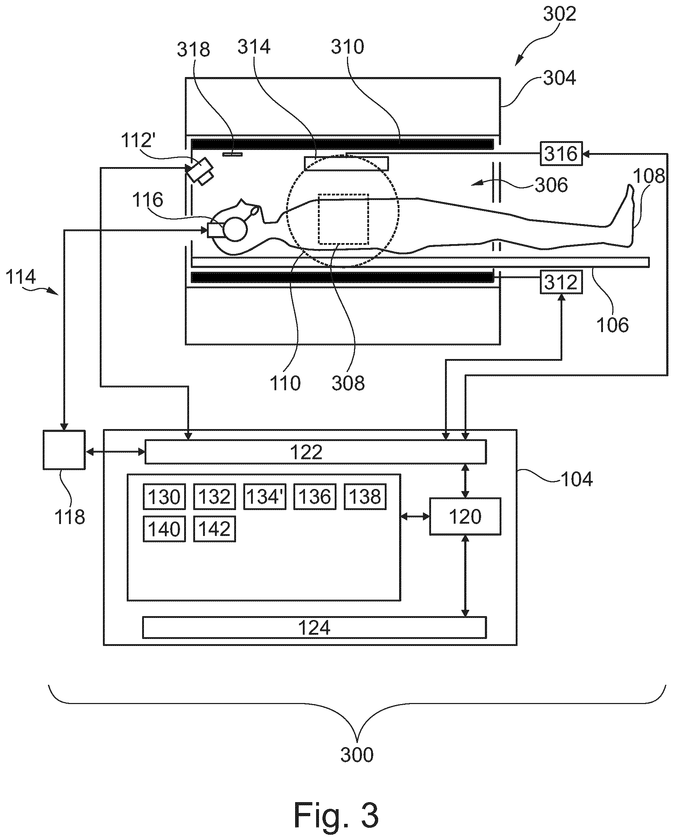

Fig. 3 illustrates a further example of the medical imaging system 300. In this example the medical scanner 102 has been replaced with a magnetic resonance imaging system 302. The magnetic resonance imaging system 302 comprises a magnet 304. The magnet 304 is a superconducting cylindrical type of magnet with a bore 306 through it. It is also possible to use both a split cylindrical magnet and a so-called open magnet. A split cylindrical magnet is similar to a standard cylindrical magnet, except that the cryostat has been split into two sections to allow access to the iso-plane of the magnet. Such magnets may for instance be used in conjunction with charged particle beam therapy. An open magnet has two magnet sections, one above the other with a space in-between that is large enough to receive a subject. The arrangement of the two magnet sections is similar to that of a Helmholtz coil. Open magnets are popular because the subject is less confined. Inside the cryostat of the cylindrical magnet there is a collection of superconducting coils.

Within the bore 306 of the cylindrical magnet 304 there is the imaging zone 110 where the magnetic field is strong and uniform enough to perform magnetic resonance imaging. A field of view 308 is shown within the imaging zone 110. The k-space data are acquired for the field of view 308. A subject 108 is shown as being supported by a subject support 106 such that at least a portion of the subject 108 is within the imaging zone 110 and the field of view 308.

Within the bore 306 of the magnet there is also a set of magnetic field gradient coils 310 which is used for the acquisition of k-space data to spatially encode magnetic spins within the imaging zone 110 of the magnet 304. The magnetic field gradient coils 310 are connected to a magnetic field gradient coil power supply 312. The magnetic field gradient coils 310 are intended to be representative. Typically, magnetic field gradient coils 310 contain three separate sets of coils for spatial encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 110 is a radio frequency coil 314 for manipulating the orientations of magnetic spins within the imaging zone 110 and for receiving radio transmissions from spins also within the imaging zone 110. The radio frequency antenna may contain multiple coil elements. The radio frequency antenna may also be referred to as a channel or antenna. The radio frequency coil 314 is connected to a radio frequency transceiver 316. The radio frequency coil 314 and radio frequency transceiver 316 may be replaced by separate transmit and receive coils and a separate transmitter and receiver. It is understood that the radio frequency coil 314 and the radio frequency transceiver 316 are representative. The radio frequency coil 314 is intended to also represent a dedicated transmit antenna and a dedicated receive antenna. Likewise, the transceiver 316 may also represent a separate transmitter and receiver. The radio frequency coil 314 may also have multiple receive/transmit elements and the radio frequency transceiver 316 may have multiple receive/transmit channels. The transceiver 316 and the gradient coil power supply 312 are shown as being connected to the hardware interface 122 of the computer system 104.

Within the bore of the magnet the remote motion sensing system in this case is a camera system 112'. There may be one or more visual markers 318 on the inside of the bore 306 which the subject 108 may look at. In this case the camera system 112' may be used for tracking the position the subject 108 is looking at. The subject specific motion pattern 132 in this example may therefore be a gaze at one or more visual markers 318. Although it is not shown in the figure, the memory may additionally store pulse sequence commands configured to control the magnetic resonance imaging system 302 to acquired the medical imaging data which in this case is k-space data 134'.

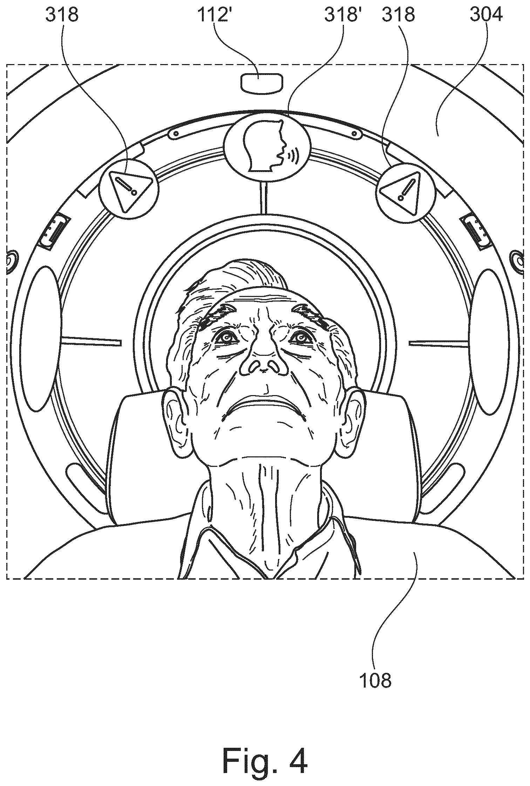

Fig. 4 shows a view from inside the bore 306 of the magnet 304. The subject 108 is positioned within view of the camera 112' and the subject 108 is able to look at one of a number of visual markers 318, 318'. For example the visual markers labeled 318 show a warning sign and could be used to indicate distress or warning by the subject 108. The visual marker 318 could be used to indicate "I have a problem and need some help." There is another visual marker 318' which shows a person talking. This could be used to call the operator and activate the subject communication system 114. The visual marker 318' could be used to indicate "I want to talk to the operator."

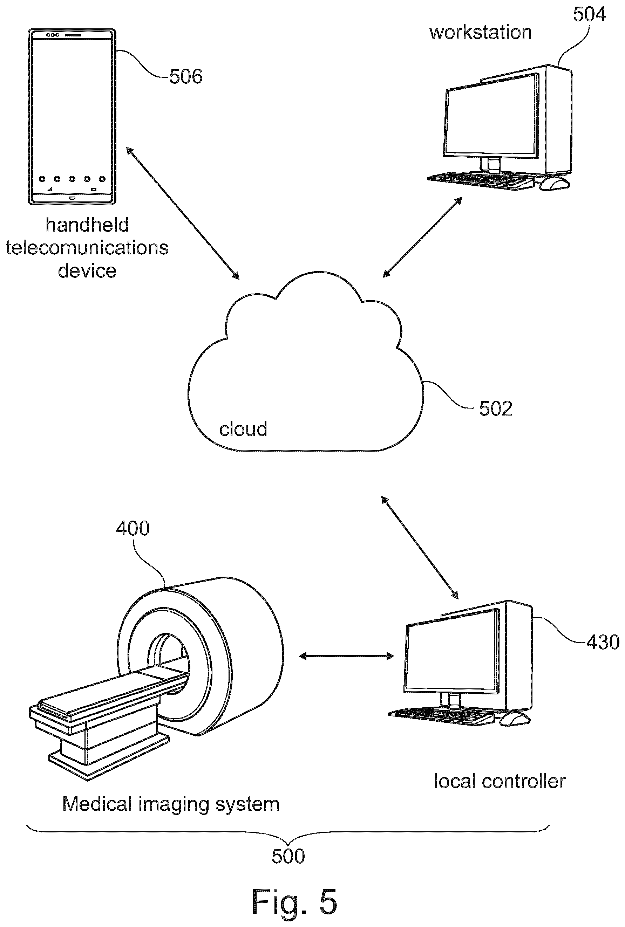

Fig. 5 shows a further example of a medical imaging system 500 that has cloud-based reconstruction. The medical imaging system 500 is shown as comprising the medical scanner 102 and the local controller or computer 104 as illustrated in Fig. 1 and also in Fig. 3. The local controller 104 may control the medical scanner 102 to acquire the medical imaging data 134. The raw medical imaging data 134 such as k-space data 134' may be transferred to a cloud-based computing system 502. Medical images may be reconstructed there and then sent back to the local controller 104 or optionally to a workstation 504 or a handheld telecommunication device 506.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit", "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational systems to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the C programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a Local Area Network (LAN) or a Wide Area Network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special-purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, WLAN connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, television screen, touch screen, tactile electronic display, Braille screen, Cathode Ray Tube (CRT), storage tube, bi-stable display, electronic paper, vector display, flat panel display, Vacuum Fluorescent (VD) display, Light-Emitting Diode (LED) displays, Electro-Luminescent Display (ELD), Plasma Display Panels (PDP), Liquid Crystal Display (LCD), Organic Light-Emitting Diode (OLED) displays, a projector, and head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical imaging system
- 102: medical scanner
- 104: computer
- 106: subject support
- 108: subject
- 110: medical imaging zone
- 112: remote motion sensing system
- 112': camera system
- 114: subject communication system
- 116: subject speaker and microphone
- 118: operator speaker and microphone
- 120: computational system
- 122: hardware interface
- 124: user interface
- 126: memory
- 130: machine executable instructions
- 132: subject specific motion pattern
- 134: medical imaging data
- 134': k-space data
- 136: pattern recognition algorithm
- 138: subject motion data
- 140: current motion pattern
- 142: alert
- 200: receive a selection of a subject specific motion pattern
- 202: acquire the medical imaging data using the medical imaging system
- 204: continually receive the subject motion data during the acquisition of the medical imaging data
- 206: continually search the motion data for the subject specific motion pattern using a pattern recognition algorithm
- 208: trigger an alert if the subject specific motion pattern is detected
- 302: magnetic resonance imaging system
- 304: magnet
- 306: bore of magnet
- 308: field of view
- 310: magnetic field gradient coils
- 312: magnetic field gradient coil power supply
- 314: radio frequency coil
- 316: transceiver
- 318: visual marker
- 318': visual marker
- 320: pulse sequence commands
- 500: medical imaging system
- 502: cloud computing system
- 504: workstation
- 506: handheld telecommunications device

## Claims

1. A method of operating a medical imaging system (100, 300, 500) configured for acquiring medical imaging data (134, 134') descriptive of a subject (108) on a subject support (106), wherein the medical imaging system comprises a remote motion sensing system (112, 112') configured for acoustically, visually, or electromagnetically acquiring subject motion data (138) descriptive of motion of the subject on the subject support, wherein the method comprises:
- receiving (200) a selection of a subject specific motion pattern (132);
- acquiring (202) the medical imaging data using the medical imaging system;
- continually (204) receiving the subject motion data during the acquisition of the medical imaging data;
- continually (206) searching the subject motion data for the subject specific motion pattern using a pattern recognition algorithm; and
- triggering (208) an alert (142) if the subject specific motion pattern is detected, wherein the alert preferably activates a subject communication system configured for opening a communication channel (114) between the subject and an operator of the medical imaging system.

2. The method of claim 1, wherein the pattern recognition algorithm comprises a trained neural network.

3. The method of claim 1 or 2, wherein the pattern recognition algorithm comprises an algorithmic pattern recognition algorithm, wherein the algorithmic pattern recognition module is preferably any one of the following: a Gaussian mixture model, an optical flow algorithm, a feature tracking algorithm, a clustering algorithm operating on output of the optical flow algorithm, and combinations thereof.

4. The method of any one of the preceding claims, wherein the subject specific motion pattern comprises a sequence of discrete subject motions.

5. The method of any one of the preceding claims, wherein the method further comprises:
- acquiring pre-acquisition subject motion data descriptive of the subject using the remote motion sensing system; and
- pre-calibrating or training the pattern recognition algorithm using the pre-acquisition subject motion data, the pre-calibrating or training being adapted to minimize false positive identification of the subject specific motion pattern by the pattern recognition algorithm.

6. The method of any one of the preceding claims, wherein the method further comprises selecting the subject specific motion pattern from a predetermined list of subject motions.

7. The method of any one of the preceding claims, wherein the method further comprises:
- labeling the subject motion data if the alert was correct or a false positive; and
- training or adjusting the pattern recognition algorithm using the labeled subject motion data.

8. The method of any one of the preceding claims, wherein the remote motion sensing system comprises a camera system (112'), wherein the subject motion data comprises optical images.

9. The method of claim 8, wherein the subject specific motion pattern comprises any one of the following: lip reading data, predetermined facial expressions, eye expressions, blinking patterns, and combinations thereof.

10. The method of claim 8 or 9, wherein the camera system is configured for eye tracking, wherein the subject motion data comprises eye motion data, wherein the subject specific motion pattern comprises eye motion data descriptive of looking at a predetermined location, wherein the predetermined location is preferably a visual marker (318, 318') or predetermined location on a video screen.

11. The method of any one of the preceding claims, wherein the remote motion sensing system comprises any one of the following: a radar, an ultrasound imaging system, a heart rate monitor, a breathing rate monitor, and combinations thereof.

12. The method of any one of the preceding claims, wherein the medical imaging system comprises a magnetic resonance imaging system (302) configured for acquiring at least a portion of the medical imaging data.

13. The method of any one of the preceding claims, wherein the medical imaging system comprises any one of the following for acquiring at least a portion of the medical imaging data: a positron emission tomography system, a single photon emission tomography system, a computed tomography system, a digital fluoroscope, a digital x-ray system, and combinations thereof.

14. A computer program product, in particular a computer readable medium, the computer program product carrying machine executable instructions (130) for execution by a computational system (120) controlling a medical imaging system (100, 300, 500), wherein execution of the machine executable instructions causes the computational system to perform the method of any one of claims 1 through 13.

15. A medical imaging system (100, 300, 500) configured for acquiring medical imaging data (134, 134') descriptive of a subject (108) on a subject support (106), wherein the medical imaging system comprises:
- a remote motion sensing system (112, 112') configured for acoustically, visually, or electromagnetically acquiring subject motion data descriptive of motion of the subject on the subject support;
- a memory (126) storing machine executable instructions (130);
- a computational system (120), wherein execution of the machine executable instructions causes the computational system to:
- receive (200) a selection of a subject specific motion pattern (132);
- acquire (202) the medical imaging data using the medical imaging system;
- continually (204) receive the subject motion data during the acquisition of the medical imaging data;
- continually (206) search the subject motion data for the subject specific motion pattern using a pattern recognition algorithm; and
- trigger (208) an alert (142) if the subject specific motion pattern is detected, wherein the alert preferably activates a subject communication system configured for opening a communication channel between the subject and an operator of the medical imaging system.
